Europäisches Patentamt

(19) European Patent Office

Office européen des brevets

(11) Publication number: **0 241 126 B1**

# EUROPEAN PATENT SPECIFICATION

(45) Date of publication of patent specification:
24.07.91 Bulletin 91/30

(51) Int. Cl.[5]: **A61K 31/19, A61K 9/16,**
**A61K 9/52**

(21) Application number: 87301825.3

(22) Date of filing: 03.03.87

(54) Pharmaceutical composition comprising aggregates of crystals of ibuprofen.

| | |
|---|---|
| The file contains technical information submitted after the application was filed and not included in this specification | (56) References cited:<br>PATENT ABSTRACTS OF JAPAN, vol. 5, no. 200 (C-84)[872], 18th December 1981; & JP-A-56 120 616 (KAKEN YAKUKAKOU K.K.) 22-09-1981 (Cat. D,A)<br>P.H. LIST et al.: "Hagers Handbuch der Pharmazeutischen Praxis", vol. 7, part A, 1971, 4th edition, pages 312-315, 728-729, Springer Verlag, Berlin, DE; |
| (30) Priority: 19.03.86 GB 8606762 | |
| (43) Date of publication of application:<br>14.10.87 Bulletin 87/42 | |
| (45) Publication of the grant of the patent:<br>24.07.91 Bulletin 91/30 | (73) Proprietor: The Boots Company PLC<br>1 Thane Road West<br>Nottingham Nottinghamshire NG2 3AA (GB) |
| (84) Designated Contracting States:<br>AT BE CH DE ES FR GB GR IT LI LU NL SE | (72) Inventor: Allan, Keith<br>30 Brookside Avenue East Leake<br>Loughborough Leicestershire (GB)<br>Inventor: Bogan, David Wesley<br>3100 Kingfish Drive<br>Shreveport Louisiana 71119 (US)<br>Inventor: Chen, Jivn-Ren<br>7614 Brookhaven<br>Shreveport Louisiana 71105 (US)<br>Inventor: Slater, Richard Michael<br>10 The Dial Cotgrave<br>Nottingham Notts. (GB) |
| (56) References cited:<br>EP-A- 0 120 587<br>EP-A- 0 172 014<br>CHEMICAL ABSTRACTS, vol. 96, no. 2, 11th January 1982, page 330, abstract no. 11617q, Columbus, Ohio, US; E.N. HIESTAND et al.: "Mechanical property changes of compacts from variation of crystallization rates of the solid", & PROC. TECH. PROGRAM: INT. POWDER BULK SOLIDS HANDL. PROCESS. 1981, 383-7<br>CHEMICAL ABSTRACTS, vol. 89, no. 4, 24th July 1978, page 407, abstract no. 30792q, Columbus, Ohio, US; & CS-A-171 463 (RUZEK) 15-02-1978 | |
| | (74) Representative: Thacker, Michael Anthony et al<br>THE BOOTS COMPANY PLC Patents Section<br>R4 Pennyfoot Street<br>Nottingham NG2 3AA (GB) |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to therapeutic agents, and in particular to pharmaceutical compositions containing the analgesic and antiinflammatory compound, ibuprofen. The compositions are therefore of value in the treatment of pain and inflammation, including rheumatoid arthritis and osteoarthritis.

Ibuprofen is (+)2-(4-isobutylphenyl)propionic acid. It is a potent and well-tolerated peripherally-acting analgesic and inflammatory compound. The present invention is concerned with pharmaceutical compositions comprising ibuprofen.

Ibuprofen is normally supplied in crystalline form. However, the form of crystalline ibuprofen normally obtained from chemical suppliers does not have very desirable flow properties, particularly after storage for a prolonged period. When required for formulation into a presentation suitable for administration to a patient, flow problems may arise in transferring the ibuprofen from one place to another, particularly during the tabletting process. It is current practice to combine ibuprofen with pharmaceutically acceptable excipients to form granules containing approximately 50-90% ibuprofen. These granules have better free-flowing properties than crystalline ibuprofen itself. However, it would be desirable to produce ibuprofen per se in a form which has superior flow properties to the form of crystalline ibuprofen normally supplied.

European Patent Application 172014 relates to a pharmaceutical composition comprising ibuprofen. The composition is in granular form and it is disclosed that in order to afford effective disintegration of the compacted granules in the dosage forms into which they are incorporated, the granules must contain a proportion of an excipient. The excipient employed in accordance with that invention is croscarmellose sodium. The granules disclosed in European Patent Application 172014 thus contain 85-99% by weight ibuprofen and 15-1% by weight croscarmellose sodium.

Japanese Patent Application No. 120616 (1981) also relates to granular pharmaceutical compositions comprising ibuprofen. The granules may be produced by heat-melting ibuprofen powder and then cooling until the melt is completely solid. The solid melt must then be taken and crushed into granules. It will be appreciated that this process may be time consuming particularly in the cooling stage. The granules formed according to this process are solid blocks of crystalline ibuprofen.

We have now found that we can provide a solid pharmaceutical composition comprising granules of ibuprofen which have advantageous free flowing properties, compression properties and formulation properties.

Accordingly the present invention provides a solid composition comprising granules consisting essentially of an aggregate of crystals of ibuprofen wherein the crystals have a median particle size in the range 5-100 μm and the granules have a porosity in the range 4-40%.

It has been found that the granules are substantially non-friable. The granules may be employed in the preparation of pharmaceutical formulations and surprisingly retain advantageous compression, tablet disintegration and dissolution characteristics.

The composition is of value in the manufacture of solid dosage therapeutic formulations. The provision of a form of ibuprofen which is free-flowing and which comprises no excipients is advantageous when it is desired to formulate ibuprofen into formulations suitable for therapeutic application as it allows the provision of a smaller tablet for a given weight of ibuprofen.

A further advantage of the invention is that solid dosage formulations may be produced by mixing a composition according to the invention with appropriate formulation excipients and compressing to form a tablet. This process avoids the need for a pre-granulation stage before the mixing stage, which has heretotore been necessary to provide a free-flowing form of ibuprofen having desirable compression characteristics.

The composition comprises granules consisting essentially of an aggregate of crystals of ibuprofen. The composition is formed by compacting crystalline ibuprofen to cause an aggregation of the crystals of ibuprofen. The median particle size of the crystals of the crystalline ibuprofen which are formed into the aggregate is in the range 5-100μm.

The flow properties of a granular composition is indicated by its percentage compressibility which is calculated from

$$\text{compressibility} = \frac{(\text{Tapped density} - \text{Bulk density})}{\text{Tapped density}} \times 100$$

The bulk density is measured by dividing the weight of the composition by the volume taken up by the composition after loosely pouring it into a container. The tapped density is a measure of the reduction in volume of the composition after settling. It may be measured by allowing a container holding the bulk composition to fall through a short distance for a given number of times, for example 200 times, and measuring the volume in order to determine the density (the weight of composition remaining the same).

In order to exhibit the desired good flow properties of a composition according to the invention, the compressibilities of the ibuprofen granules should be less than 28%, preferably 5-20%. In contrast, unprocessed ibuprofen crystals typically have a compressibility of 36% or greater indicating poor flow properties.

The bulk density of the preferred granule is in the range 0.3-0.9 $g/cm^3$, conveniently 0.3-0.7 $g/cm^3$. Most suitably the granules have a bulk density in the range 0.3-0.6 $g/cm^3$. A suitable range of values for the tapped density of the granules is 0.35-1.0 $g/cm^3$, preferably 0.4-0.8 $g/cm^3$ and more preferably 0.5-0.7 $g/cm^3$.

The angle of repose is another indication of the free-flowing characteristics of granular compositions. The angle of repose is taken to be the angle the outer surface of a pile of granules poured onto a horizontal surface makes with the surface. Advantageously the angle of repose of a composition according to the invention is less than 55°, which shows the granules to have valuable free-flowing characteristics. Preferred granules have an angle of repose in the range 20°-45°.

Suitably the sizes of the granules are such that, on formulation in a solid dosage form, little or no segregation from excipients occurs. Suitably the granules have a median size in the range 50-2000 µm, preferably 200-800 µm, for example 250-650µm.

The granules of ibuprofen have advantageously high strength i.e. they are not readily broken down into smaller particles on further processing. One method of assessing strength of the granules is measurement of friability. The friability may be measured by tumbling the granules with glass beads for approximately 10 minutes and then screening to separate the granules from the fines. The percentage friability is equal to the percentage weight loss. Suitably the granules have a friability of less than 10%, in particular less than 5%, preferred granules leaving a friability of less than 2%.

The degree of compaction of crystalline ibuprofen into the aggregate material may be measured by the porosity of the granule. The granule porosity is the percentage volume of gaseous phase within each granule. The porosity is in the range 4-40%. A porosity in this range confers advantageous disintegration and dissolution properties to formulations containing the granules. A more preferred porosity is in the range 5-20%. Especially preferred granules have a porosity in the range 6-12%. The porosity measurements referred to herein are measured by mercury intrusion porosimetry using a pressure of one atmosphere.

The granules of a composition according to the invention suitably have a surface area of greater than 0.3 $m^2/g$, particularly in the range 0.4-1.0 $m^2/g$. A high surface area leads to advantages in dissolution characteristics.

The compositions of this invention may be formulated with suitable excipients to manufacture solid dosage formulations of ibuprofen suitable for therapeutic application. In particular the compositions may be compounded with lubricants, surfactants, disintegrants, binders and diluents and compressed into a solid dosage presentation. The solid dosage presentation may comprise any desired presentation for example capsules, tablets, lozenges, suppositories or implants. It is preferred to provide the solid dosage presentation in the form of tablets. Lubricants may include stearic acid, magnesium stearate, calcium stearate, or other metallic stearates and talc. Surfactants may include sodium lauryl sulphate and polysorbate 20. Tablet disintegrants may include corn starch, sodium starch glycollate and sodium croscarmellose. Binders may include polyvinylpyrrolidone, gelatin, starches or microcrystalline cellulose. Diluents may include lactose, sodium chloride, dextrins, calcium phosphate, calcium sulphate and sucrose. Although such formulation excipients may comprise a significant proportion of the formulation, for example up to 50%, in order to reduce the size of a solid dosage presentation, suitably the formulation comprises less than 40% excipient, for example 15-35% excipient.

Formulations may, if desired, be coated with any conventional coating, for example a film coating or a sugar coating.

If desired a composition according to the invention may be combined with any conventional excipient providing sustained release properties, preferably in the form of a coating of sustained release material around the granule. The coated granules may be compressed to form a tablet or other suitable solid dosage form. Examples of suitable sustained release materials include natural gums, synthetic gums and modified cellulose derivatives.

A formulation may comprise 50-95% by weight of a composition according to the invention, preferably 60-90% and more preferably 65-85% by weight.

Each unit dose composition suitably contains from 50-1200 mg of ibuprofen, preferably from 200 to 800 mg. Formulations may be prepared by mixing a composition according to the invention with a pharmaceutically acceptable carrier as hereinabove disclosed. Preferably they are filled into a capsule, or the blend may be compressed to form a tablet.

The invention provides in a further aspect a process for preparing a solid pharmaceutical composition comprising granules consisting essentially of an aggregate of crystals of ibuprofen comprising compacting crystalline ibuprofen wherein the crystals have a median particle size in the range 5-100 μm and the granules have a porosity in the range 4-40% to cause an aggregation of the crystalline ibuprofen to form an aggregate material, comminuting the aggregate material and selecting granules of the desired size.

A preferred process comprises compacting crystalline ibuprofen having crystals with a median particle size of 5 to 100μm to cause an aggregation of the crystals to form an aggregate material, comminuting the aggregate material and selecting the granules having a median particle size in the range 50-2000μm and a bulk density in the range 0.3 to 0.9 g/cm³.

Conveniently the granules are produced by sizing or comminuting the aggregate material for example by sieving, chopping, milling, grinding or cutting into lengths. If desired, the stages of comminuting the aggregate material and selecting the granules may occur simultaneously, e.g. by passing the aggregate material through a sieve.

Crystalline ibuprofen used in the preparation of the granules may be dry. For example, dry ibuprofen may be taken after the drying process in the production of ibuprofen and placed into a hopper arranged to feed material into a suitable granulating apparatus. Alternatively, crystalline ibuprofen may contain a proportion of liquid. Crystalline ibuprofen containing a proportion of liquid is referred to herein as damp ibuprofen.

Any liquid which wets ibuprofen may be used to produce the damp ibuprofen, for example water, preferably with a surfactant. Preferably however, a non-aqueous liquid is employed, for example a ketone, in particular methyl isobutyl ketone and acetone ; an alcohol, particularly methanol and isopropanol ; or dichloromethane. A more preferred liquid is a hydrocarbon, especially a petroleum distillation fraction, for example hexane. Suitably, crystalline ibuprofen may contain up to 50% by weight of a liquid, advantageously up to 30%, preferably up to 20% and in particular 5-15% by weight. When a liquid is used, a process according to the invention should include a drying stage to reduce the liquid content to less than 1%. Either the aggregate material or the granule may be dried, or if desired, both the aggregate material and the granule may be dried.

In a preferred aspect of this invention, damp ibuprofen is employed in a wet granulation process. Damp ibuprofen may be obtained by wetting the dry ibuprofen obtained at the end of the ibuprofen production process. Preferably however, the crystalline ibuprofen used in the preparation of the granules is the damp material obtained prior to the drying stage in any of the commercial processes to prepare ibuprofen. Taking the material at this point in the process, thereby avoiding the final drying stage, provides a significant reduction in the cost of providing a free-flowing form of ibuprofen.

A preferred process according to the invention comprises compacting crystalline ibuprofen containing up to 50% by weight of a liquid to form an aggregate material, drying the aggregate material, comminuting the aggregate material, and selecting the granules having a median particle size in the range 50-2000μm and a bulk density in the range 0.3 to 0.9 g/cm³. Alternatively, after compacting the crystalline ibuprofen to form an aggregate material, the aggregate material is comminuted and the granules having the above disclosed particle size and bulk density are selected and then dried.

A composition according to the invention may be prepared by compacting crystalline ibuprofen by a roller compactor which may comprise several rollers or by compacting between a roller and a flat bed. Preferably crystalline ibuprofen is compacted between two counter-rotating rollers. In this case, the crystalline ibuprofen is fed between the rollers by a screw arrangement from a hopper. The rollers are arranged to counter-rotate to urge the crystalline ibuprofen between the rollers. The screw feed rate, the roller speed of each roller and the roller pressure on the crystalline ibuprofen passing between the rollers may be varied to provide an aggregate material having a desired density. The aggregate material is broken down into granules, this breakdown may further be aided by a comminutor fitted at the end of the compactor.

Altenatively, the crystalline ibuprofen may be compacted twice, initially as it passes between first and second rollers, and subsequently as it passes between second and third rollers of a set of three rollers, the first and third rollers being arranged to rotate in the same direction, the second roller being arranged to rotate in the opposite direction. In this case, the crystalline ibuprofen is fed between the first and second rollers from a hopper. The layer of aggregate material adhering to the second roller transfers to the third roller and is scraped off by a scraper bar. Again, the roller speed of each roller and the roller pressure on the crystalline ibuprofen passing between the rollers may be varied as desired to produce a suitable aggregate material. The aggregate material is broken down to a granular material and dried by any convenient process, for example by drying in trays.

In a preferred process according to the invention, crystalline ibuprofen containing up to 50% by weight of a liquid may be compacted by extrusion through an extruding apparatus. Preferably crystalline ibuprofen is extruded by urging it between two rollers, one roller having a solid surface and the other roller being hollow and having extruding apertures which extend radially from the hollow interior to the external surface of the roller, and extruding the crystalline ibuprofen through the apertures into the interior of the hollow roller. One such form of apparatus in which this may be carried out is the roller extruder. The extruding apertures in the hollow roller are located throughout the length of the roller ; they may be of any desired size to produce a suitable aggregate material. The rollers are set at a desired distance apart and at a desired speed of rotation to provide adequate extrusion to produce an aggregate material having a desired porosity. The extrudate which is the desired aggregate material may be scraped off by a scraper bar. The extrudate may be further broken down by a screening device which comprises a mesh or meshes designed to screen the extrudate down to the required size. Any liquid-containing granules may be dried by any suitable drying process, for example in trays at ambient temperature, either before or after the screening. In a preferred extrusion process according to the invention crystalline ibuprofen containing up to 50% by weight of a liquid is compacted by urging it between the two rollers of a roller extruder (as described above), extruding part of the crystalline ibuprofen through the extruding apertures into the interior of the hollow roller and collecting a first aggregate material, collecting a second aggregate material which is prepared by compacting the remaining part of the crystalline ibuprofen between the rollers, mixing said first and second aggregate materials, comminuting the mixture and selecting the granules having a median particle size in the range 50-2000µm and a bulk density in the range 0.3 to 0.9 g/cm³. The drying stage may occur before or after comminution of the aggregate material.

Advantageously crystalline ibuprofen may also be compacted by conveying crystalline ibuprofen containing up to 50% by weight of a liquid by conveying screws through an extruder and extruding the crystalline ibuprofen through an extruder plate. The extruder plate comprises extruding apertures extending through the thickness of the plate. The plate may be of any thickness, for example 0.5mm-20mm. The extruding apertures may vary in diameter but preferably are from 0. 5mm-10mm.

Alternatively, crystalline ibuprofen may be compacted by kneading with kneading paddles as it is conveyed by conveying screws through an extruder. If desired, the aggregate material obtained by kneading may also be passed through an extruder plate which may be affixed to an end portion of the extruder. The extruder plate comprises a plate having extruding apertures extending through the thickness of the plate (as described above). An extruder suitable for use in the kneading process comprises kneading paddles and conveying screws, and is either a single screw extruder or a twin screw extruder. The speed of rotation of the screws and the angles at which the kneading paddles are arranged in the extrusion chamber may be varied to vary the properties of the aggregate material and to provide a granule suitably having a bulk density in the range 0.3-0.9 g/cm³. In addition, where the extruder is provided with an extruder plate, a chopper comprising chopping blades may optionally be provided to cut the aggregate material extruded through the extruder plate into lengths. Where dry crystalline ibuprofen is passed into the extruder, a liquid may be added to the crystalline ibuprofen as it passes along the length of the extruder.

In a further process to form a composition comprising a granule consisting essentially of an aggregate of ibuprofen crystals, crystalline ibuprofen containing up to 50% by weight liquid may be compacted as it rotates under the action of rotary arms in a granulation bowl. For example, this may be carried out in a high shear granulator in which damp crystalline ibuprofen is stirred and densified for a sufficient time to provide an aggregate material having desired properties.

The granules produced by any of the processes described above may be screened to obtain granules of the desired size.

The invention is illustrated by the following non-limitative examples.

In the Examples, the properties of the ibuprofen granules are defined according to the following tests.

### 1. Friability Test

10 g of ibuprofen granules previously sieved to produce size fractions greater than 355 µm are tumbled for ten minutes at 30 rpm in a friabulator with ten 3 mm glass beads. The sample is resieved on a 355 µm sieve and the weight of the remaining granules measured. The percentage friability is equal to the percentage weight loss resulting from tumbling with the glass beads. The percentage friability is a measure of the strength of a granule.

### 2. Angle of Repose

The angle of repose is taken to be the angle the outer surface of a pile of ibuprofen granules poured onto a horizontal surface makes with the surface. It is measured by carefully pouring the granules onto a level surface, and then measuring the angle between the outer surface of the pile and the horizontal surface.

### 3. Tapped Density

Approximately 200 cm³ of the granular material is weighed into a graduated glass cylinder. The cylinder is allowed to fall from a height of 2.5 cm at a rate of 30 times per minute by means of an eccentric cam. After the cylinder is dropped 200 times, the volume of the granular material is measured and the tapped density calculated.

## 4. Bulk Density

The bulk density is calculated by dividing the weight of the composition by the volume of the composition after loosely pouring it into a container.

## 5. Compressibility

The compressibility is calculated from

$$\% \text{ Compressibility} = \frac{(\text{Tapped Density-Bulk Density})\text{x } 100}{\text{Tapped Density}}$$

## 6. Porosity

This is measured by mercury intrusion porosimetry wherein mercury is caused to enter the sample at a pressure of one atmosphere. The percentage porosity is measured by calculating
a) the density of the granule ;
b) calculating the relative density from the equation

$$\text{relative density} = \frac{\text{density}}{\text{true density}}$$

wherein the true density is calculated by dividing the weight of the sample by the volume of the sample without voids and pores ;
c) calculating % porosity by the equation

$$\% \text{ porosity} = (1\text{-relative density}) \times 100$$

## Example 1

Crystalline ibuprofen wetted with 11.5% w/w of a petroleum distillation fraction containing approximately 50% n-hexane, sold under the trade name Exsol hexane available from Esso, was fed between twin rollers of a roller extruder (Alexanderwerk Extruder Model GA65) from a hopper. One roller is solid and the other is hollow with 1 mm extruding apertures extending radially from the hollow interior to the external surface, along its length. The damp material passing between the rotating rollers was extruded through the 1 mm apertures into the centre of the hollow roller and scraped off by a scraper bar. The granules were dried in trays at ambient temperature to reduce the liquid content to less than 0.5% w/w. They were found to have the following properties :-

| | |
|---|---|
| Median particle size = | 721 μm |
| Bulk density = | 0.43 g/cm$^3$ |
| Tapped density = | 0.52 g/cm$^3$ |
| Compressibility = | 17.3% |
| Angle of Repose = | 42° |
| Friability = | 8% |
| Porosity = | 15.8% |

The granules produced had a fine structure. On magnification it could be seen that the granules were composed of agglomerates of densely packed crystals of the original crystalline material.

## Example 2

Crystalline ibuprofen wetted with 8.8% w/w of a petroleum distillation fraction containing approximately 50% n-hexane, sold under the trade name Exsol hexane available from Esso, was fed between twin rollers of a roller extruder (Alexanderwerke Extruder Model G1/100/160S) from a hopper. One roller is solid and the other is hollow with 1mm extruding apertures extending radially from the hollow interior to the external surface. The damp material passing between the rotating rollers was extruded through the 1mm apertures into the centre of the hollow roller and scraped off by a scraper bar. The damp extrudate was dried in a Calmic fluid bed drier to reduce the liquid content to less than 0.5% w/w. The dried extrudate was broken down to granules on a 630μm screen fitted in an Alexanderwerk RFG150 sieve and found to have the following properties :-

Median particle size = 420 μm
Bulk density = 0.54 g/cm$^3$
Tapped density = 0.65 g/cm$^3$
Compressibility = 17.5%
Angle of Repose = 40°
Friability = 1.0%
Surface area = 0.64 m$^2$/g
Porosity = 9.5%

Example 3

Crystalline ibuprofen wetted with 9.1% w/w of a petroleum distillation fraction containing approximately 50% n-hexane, sold under the trade name Exsol hexane available from Esso, was fed between twin rollers of the roller extruder described in Example 2 from a hopper. The damp extrudate was broken down to granules on a 800μm screen fitted in an Alexanderwerk RFG150 sieve. The granules were dried in an Aeromatic AES 5.5 fluid bed drier to reduce the liquid content to less than 0.5% w/w. They were found to have the following properties :-

Median particle size = 396 μm
Bulk density = 0.53 g/cm$^3$
Tapped density = 0.62 g/cm$^3$
Compressibility = 13.8%
Angle of Repose = 40°
Friability = 1.1%
Surface area = 0.95 m$^2$/g
Porosity = 6%

Example 4

Crystalline ibuprofen containing 11.0% w/w of a petroleum distillation fraction under the trade name Solvesso 150 available from Esso, was fed between the rollers of a laboratory triple roller compactor (Pascal Roller). The damp material was fed from a hopper between the first and second rollers, the layer of material adhering to the second roller was transferred to the third roller and was scraped off by a scraper bar to produce an aggregate material of 0.5 mm thickness. The aggregate material was dried in trays at ambient temperature for 24 hours and then at 40°C for four hours to reduce the liquid content to less than 0.5% w/w. The aggregate material was broken down on a sieve to produce granules having the following properties :-

Median particle size = 601 μm
Bulk density = 0.44 g/cm$^3$
Tapped density = 0.50 g/cm$^3$
Compressibility = 12.6%
Friability = 3.8%

Example 5

Crystalline ibuprofen containing 11.0% w/w of a petroleum distillation fraction under the trade name Solvesso 150 available from Esso, was fed between the rollers of a twin roller compactor (Alexanderwerk Roller Compactor Model WP50N/75) from a hopper by twin screws. The roller compactor was fitted with a comminutor fitted with a 2.5 mm screen. The granules produced after the crystalline ibuprofen had passed between the rollers, through the screen of the comminutor and through a dryer were found to have the following properties :-

Median particle size = 759 μm
Bulk density = 0.60 g/cm$^3$
Tapped density = 0.65 g/cm$^3$
Compressibility = 7.7%

7

| | |
|---|---|
| Angle of Repose = | 32° |
| Friability = | 0.1% |

Example 6

Dry ibuprofen crystals were passed through the twin rollers of the twin roller compactor described in Example 5. The aggregate material obtained was broken down to granules by a comminutor fitted with a 2.5 mm screen to give granules having the following properties :-

| | |
|---|---|
| Median particle size = | 536 μm |
| Bulk density = | 0.48 g/cm³ |
| Tapped density = | 0.57 g/cm³ |
| Compressibility = | 15.8% |
| Angle of Repose = | 38° |
| Friability = | 0.2% |
| Surface area = | 1.09 m²/g |

Example 7

Crystalline ibuprofen wetted with 11% of a petroleum distillation fraction under the trade name Solvesso 150 available from Esso, was passed from a hopper through a twin screw kneading machine fitted with elliptical paddles within the screw barrels. The granules produced after passing through the kneading machine were dried on trays at ambient temperatures to reduce the liquid content to less than 0.5% w/w. The dry granules before optional sieving were found to have the following properties :-

| | |
|---|---|
| Median particle size = | 505 μm |
| Bulk density = | 0.42 g/cm³ |
| Tapped density = | 0.54 g/cm³ |
| Compressibility = | 22.2% |
| Angle of Repose = | 44° |
| Friability = | 1.8% |

Example 8

Crystalline ibuprofen wetted with 11% of a petroleum distillation fraction containing approximately 50% n-hexane under the trade name Exsol hexane available from Esso, was passed from a hopper through a twin screw kneading machine fitted at the end of the barrels with an extrusion plate having 1.7 mm holes. The extrudate was broken down into granules by passing through a scraped surface sieve fitted with a 1 mm screen. The granules were vacuum dried at 40°C to reduce the liquid content to less than 0.5% w/w and found to have the following properties :-

| | |
|---|---|
| Median particle size = | 530 μm |
| Bulk density = | 0.48 g/cm³ |
| Tapped density = | 0.60 g/cm³ |
| Compressibility = | 20% |
| Angle of Repose = | 45° |
| Friability = | 1.5% |
| Porosity = | 15% |
| Surface area = | 0.98 m²/g |

Example 9

Crystalline ibuprofen containing 10.8% w/w of a petroleum distillation fraction containing approximately 50% n-hexane under the trade name Exsol hexane available from Esso, was fed from a hopper into the twin conveying screws of a Fuji Pandal Extruder and was extruded through a die plate having 1.5 mm holes. The extrudate was broken down into granules by passing through a scraped surface sieve fitted with a 1 mm screen. The granules were tray dried at room temperature to reduce the liquid content to less than 0.5% w/w. They were found to have the following properties :-

| | |
|---|---|
| Median particle size = | 481 μm |
| Bulk density = | 0.42 g/cm³ |
| Tapped density = | 0.53 g/cm³ |

| Compressibility = | 21.5% |
|---|---|
| Friability = | 4% |

## Example 10

180 ml of a mixture of 4 parts by weight dichloromethane and 1 part by weight ethanol was added to 8 kg crystalline ibuprofen whilst stirring in a high speed bowl granulator. On completion of the addition of the liquid, the mixture was stirred and agitated at high speed with intensifier blades for three minutes. The resultant granules were tray dried at 30°C to reduce the liquid content to less than 0.5% w/w before being passed through a 1000 μm sieve. The granules were found to have the following properties :-

| Median particle size = | 350 μm |
|---|---|
| Bulk density = | 0.60 g/cm³ |
| Tapped density = | 0.71 g/cm³ |
| Compressibility = | 15.5% |

## Example 11

Dry ibuprofen crystals were passed through the twin rollers of a Fitzpatrick Chilsonator and sized through a 12 mesh screen using a Jackson Crockatt Granulator to produce granules of ibuprofen. The ibuprofen granules (78.9%) were blended with spray dried lactose (13.6%), hydroxypropyl cellulose (3.3%), croscarmellose sodium NF (3.3%), and magnesium stearate (0.9%). The resultant mixture was compressed to form tablets containing 600 mg ibuprofen. The disintegration characteristics were measured using USP disintegration Apparatus (1985, Vol. XXI, p.1242) and found to be 269 seconds. The dissolution characteristics were measured using USP Apparatus 1 (1985, Vol. XXI, p.1243) using a stirrer speed of 150 rpm with the tablets placed in baskets in pH 7.2 buffer solution. 50% of the ibuprofen dissolved in $8\frac{1}{2}$ minutes.

## Example 12

780 g of ibuprofen granules prepared according to Example 1 were blended with 168 g of spray dried lactose, 20 g microcrystalline cellulose, 20 g sodium croscarmellose, 10 g magnesium stearate and 2 g colloidal silicon dioxide. The resultant mixture was compressed to form tablets containing 300 mg ibuprofen. The dissolution characteristics were measured using 1985 USP vol. XXI, page 1243, Apparatus 1, using a stirrer speed of 150 rpm with the tablets placed in baskets in pH 7.2 buffer solution. The tablets disintegrated within 35 seconds in water, and 50% dissolved in 4 minutes and 90% in 13 minutes in pH 7.2 buffer solution.

## Example 13

A formulation was prepared comprising the following ingredients :-

| | % w/w |
|---|---|
| Ibuprofen composition from Example 2 | 78 |
| Microcrystalline cellulose | 12 |
| French chalk | 2.07 |
| Tricalcium phosphate | 5.2 |
| Croscarmellose sodium | 1.95 |
| Stearic acid | 0.78 |

The ibuprofen composition was blended with the excipients and the resultant mixture compressed to form tablets containing 600 mg ibuprofen. The disintegration characteristics were measured using a BP 1973 disintegration apparatus without discs. The dissolution characteristics were measured using 1985 USP, Vol. XXI, page 1244, Apparatus 2 using a paddle speed of 50 rpm with pH 7.2 buffer. The tablets had a hardness of 12 kp, they disintegrated within 30 seconds, and 80% of the ibuprofen dissolved in 22 minutes.

### Example 14

A formulation containing 600 mg ibuprofen was prepared in the same manner as described in Example 13 but using the following ingredients :-

|  | % w/w |
|---|---|
| Ibuprofen composition from Example 2 | 78 |
| Microcrystalline cellulose | 19.14 |
| Croscarmellose sodium | 1.95 |
| Colloidal silicon dioxide | 0.13 |
| Stearic acid | 0.78 |

The disintegration and dissolution characteristics were measured in the same way as described in Example 13. The tablets had a hardness of 15 kp, they disintegrated within 30 seconds, and 80% of the ibuprofen dissolved in 20 minutes.

### Example 15

A formulation containing 600 mg ibuprofen was prepared in the same manner as described in Example 13 but using the following ingredients :-

|  | % w/w |
|---|---|
| Ibuprofen composition from Example 2 | 78 |
| Microcrystalline cellulose | 13 |
| Lactose | 3.13 |
| Tricalcium phosphate | 3.13 |
| Croscarmellose sodium | 1.96 |
| Stearic acid | 0.78 |

The disintegration and dissolution characteristics were measured in the same way as described in Example 13. The tablets had a hardness of 13 kp, they disintegrated within 45 seconds, and 80% of the ibuprofen dissolved in 19 minutes.

### Example 16

A formulation containing 600 mg ibuprofen was prepared in the same manner as described in Example 13 but using the following ingredients :-

|                                    | % w/w |
| ---------------------------------- | ----- |
| Ibuprofen composition from Example 2 | 78    |
| Microcrystalline cellulose         | 17.17 |
| Croscarmellose sodium              | 1.96  |
| French chalk                       | 2.09  |
| Stearic acid                       | 0.78  |

The disintegration and dissolution characteristics were measured in the same way as described in Example 13. The tablets had a hardness of 14 kp, they disintegrated within 45 seconds, and 80% of the ibuprofen dissolved in 18 minutes.

Formulations according to Examples 17-21 were made up in a similar manner to that described in Example 13 using the ingredients shown in Table 1.

The disintegration and dissolution characteristics of Examples 17-21 were found to be as shown in Table 2. The disintegration characteristics were measured using a BP 1973 disintegration apparatus without discs. The dissolution characteristics were measured using 1985 USP, Vol. XXI, p.1244, Apparatus 2 using a paddle speed of 50 rpm with pH 7.2 buffer.

EP 0 241 126 B1

### Table 1

| Example | 17 | 18 | 19 | 20 | 21 |
|---|---|---|---|---|---|
| Example from which ibuprofen granule is obtained | Ex.2 | Ex.3 | Ex.5 | Ex.6 | Ex.8 |
| **Formulation Ingredients (% w/w)** | | | | | |
| Ibuprofen granule | 50 | 78 | 79.2 | 79.2 | 78 |
| Microcrystalline cellulose | 40 | 2 | - | - | 2 |
| Croscarmellose sodium | 4.6 | 2 | 3.25 | 3.25 | 2 |
| Lactose | - | 16.8 | 13.4 | 13.4 | 16.8 |
| Stearic acid | 0.5 | - | - | - | - |
| Magnesium stearate | - | 1 | 0.95 | 0.95 | 1 |
| Colloidal silicon dioxide | - | 0.2 | - | - | 0.2 |
| low-substituted hydroxy propyl cellulose | - | - | 3.2 | 3.2 | - |
| French chalk | 4.9 | - | - | - | - |

EP 0 241 126 B1

### Table 2

| Example | 17 | 18 | 19 | 20 | 21 |
|---|---|---|---|---|---|
| Tablet Size | 600 mg | 300 mg | 300 mg | 300 mg | 300 mg |
| Disintegration Time (Secs) | 45 | 22 | 26 (3) | 28 (3) | 32 |
| $T_{50}$(1) | - | 6.5 | 7.5 | 4.5 | 4.5 |
| $T_{90}$(2) | - | 17.5 | 23 | 14 | 16 |

Notes (1)   $T_{50}$ is the time taken for 50% of the ibuprofen to dissolve (minutes)

(2)   $T_{90}$ is the time taken for 90% of the ibuprofen to dissolve (minutes)

(3)   Dissolution measured by 1985 USP, Vol. XXI, p. 1243, Apparatus 1

EP 0 241 126 B1

## Claims

### Claims for the following Contracting States : BE, CH, DE, FR, GB, IT, LI, LU, NL, SE

1. A solid pharmaceutical composition comprising granules consisting essentially of an aggregate of crystals of ibuprofen wherein the crystals have a median particle size in the range 5-100μm and the granules have a porosity in the range 4-40%.

2. A composition according to claim 1 wherein the granules have a bulk density in the range 0.3-0.7 g/cm³ and a tapped density in the range 0.4 to 0.8 g/cm³.

3. A composition according to either one of the preceding claims wherein the granules have a median particle size in the range 50-2000μm.

4. A solid pharmaceutical formulation comprising 50-95% by weight of a composition according to any one of claims 1 to 3 together with a pharmaceutically acceptable carrier.

5. A formulation according to claim 4 wherein the pharmaceutically acceptable carrier comprises one or more of stearic acid, cellulose or cellulose derivatives, disaccharide, colloidal silicon dioxide, croscarmellose sodium, magnesium stearate, french chalk, tricalcium phosphate.

6. A formulation according to either one of claims 4 and 5 wherein the granules are coated with a sustained release carrier to form a sustained release formulation.

7. A process for preparing a solid pharmaceutical composition comprising granules consisting essentially of an aggregate of crystals of ibuprofen wherein the crystals have a median particle size in the range 5-100μm and the granules have a porosity in the range 4-40%, comprising compacting crystalline ibuprofen to cause an aggregation of the crystalline ibuprofen to form an aggregate material, comminuting the aggregate material and selecting the granules having a desired size.

8. A process according to claim 7 wherein comminuting the aggregate material and selecting the granules occur simultaneously.

9. A process according to either one of claims 7 and 8 wherein the aggregate material is comminuted by sieving to produce the granules.

10. A process according to any one of claims 7 to 9 wherein the crystalline ibuprofen contains up to 50% by weight of a liquid, and the liquid is removed from the aggregate material or the granules by drying.

11. A process according to claim 10 wherein the liquid comprises a hydrocarbon.

12. A process according to any one of claims 7 to 11 in which the crystalline ibuprofen is compacted
a) between two counter-rotating rollers to form the aggregate material ;
b) initially as it passes between first and second rollers and subsequently as it passes between second and third rollers of a set of three rollers, the first and third rollers being arranged to rotate in the same direction, the second roller being arranged to rotate in the opposite direction, and the aggregate material being collected after compaction between the second and third rollers.

13. A process according to either one of claims 10 and 12 wherein the crystalline ibuprofen is compacted by
a) urging the crystalline ibuprofen between two rollers, one roller having a solid surface and the other roller being hollow and having extruding apertures which extend radially from the hollow interior to the external surfare of the roller, and extruding the crystalline ibuprofen through the extruding apertures into the interior of the hollow roller ;
b) conveying the crystalline ibuprofen by conveying screws through an extruder, and extruding the crystalline ibuprofen through an extruder plate, the extruder plate comprising extruding apertures extending through the thickness of the plate ; or
c) kneading the crystalline ibuprofen with kneading paddles as it is conveyed by conveying screws through an extruder to form an aggregate material.

### Claims for the following Contracting States : AT, GR, ES

1. A process for preparing a solid pharmaceutical composition comprising granules consisting essentially of an aggregate of crystals of ibuprofen wherein the crystals have a median particle size in the range 5-100μm and the granules have a porosity in the range 4-40%., comprising compacting crystalline ibuprofen to cause an aggregation of the crystalline ibuprofen to form an aggregate material, comminuting the aggregate material and selecting the granules having a desired size.

2. A process according to claim 1 wherein comminuting the aggregate material and selecting the granules

14

occur simultaneously.

3. A process according to either one of claims 1 and 2 wherein the aggregate material is comminuted by sieving to produce the granules.

4. A process according to any one of claims 1 to 3 wherein the crystalline ibuprofen contains up to 50% by weight of a liquid, and the liquid is removed from the aggregate material or the granules by drying.

5. A process according to claim 4 wherein the liquid comprises a hydrocarbon.

6. A process according to any one of claims 1 to 5 in which the crystalline ibuprofen is compacted

a) between two counter-rotating rollers to form the aggregate material ;

b) initially as it passes between first and second rollers and subsequently as it passes between second and third rollers of a set of three rollers, the first and third rollers being arranged to rotate in the same direction, the second roller being arranged to rotate in the opposite direction, and the aggregate material being collected after compaction between the second and third rollers.

7. A process according to either one of claims 4 and 5 wherein the crystalline ibuprofen is compacted by

a) urging the crystalline ibuprofen between two rollers, one roller having a solid surface and the other roller being hollow and having extruding apertures which extend radially from the hollow interior to the external surface of the roller, and extruding the crystalline ibuprofen through the extruding apertures into the interior of the hollow roller ;

b) conveying the crystalline ibuprofen by conveying screws through an extruder, and extruding the crystalline ibuprofen through an extruder plate, the extruder plate comprising extruding apertures extending through the thickness of the plate ; or

c) kneading the crystalline ibuprofen with kneading paddles as it is conveyed by conveying screws through an extruder to form an aggregate material.

8. A process to prepare a solid pharmaceutical formulation comprising 50-95% by weight of a composition comprising granules consisting essentially of an aggregate of crystals of ibuprofen wherein the crytals have a median particle size in the range 5-100μm and having a porosity in the range 4-40% together with a pharmaceutically acceptable carrier wherein the composition is mixed with the pharmaceutically acceptable carrier and compressed into a solid dosage form.


## Patentansprüche

### Patentansphrüche für folgenden Vertragsstaaten : BE, CH, DE, FR, GB, IT, LI, LU, NL, SE

1. Feste pharmazeutische Zusammensetzung, welche Körnchen umfaßt, die im wesentlichen aus einem Aggregat von Kristallen von Ibuprofen bestehen, worin die Kristalle eine mittlere Teilchengröße im Bereich von 5 bis 100 μm aufweisen und die Körnchen eine Porosität im Bereich von 4 bis 40 % aufweisen.

2. Zusammensetzung nach Anspruch 1, worin die Körnchen eine Schüttdichte im Bereich von 0,3 bis 0,7 g/cm³ und eine Rütteldichte im Bereich von 0,4 bis 0,8 g/cm³ aufweisen.

3. Zusammensetzung nach einem der beiden vorangehenden Ansprüche, worin die Körnchen eine mittlere Teilchengröße im Bereich von 50 bis 2000 μm aufweisen.

4. Feste pharmazeutische Formulierung, welche 50 bis 95 Gew.-% einer Zusammensetzung gemäß irgendeinem der Ansprüche 1 bis 3 zusammen mit einem pharmazeutisch annehmbaren Träger umfaßt.

5. Formulierung nach Anspruch 4, worin der pharmazeutisch annehmbare Träger eine oder mehrere Verbindungen aus der Gruppe Stearinsäure, Cellulose oder Cellulose-Derivate, Disaccharide, kolloidales Silicium-Dioxid, Croscarmellose-Natrium, Magnesium-Stearat, Talkum und Tricalciumphosphat umfaßt.

6. Formulierung nach einem der beiden Ansprüche 4 und 5, worin die Körnchen mit einem Träger für verzögerte Freisetzung der Wirkstoffe unter Bildung einer Formulierung mit verzögerter Freisetzung der Wirkstoffe beschichtet sind.

7. Verfahren zur Herstellung einer festen pharmazeutischen Zusammensetzung, die Körnchen umfaßt, die im wesentlichen aus einem Aggregat aus Kristallen von Ibuprofen bestehen, worin die Kristalle eine mittlere Teilchengröße im Bereich von 5 bis 100 μm aufweisen und die Körnchen eine Porosität im Bereich von 4 bis 40 % aufweisen, wobei das Verfahren folgende Schritte umfaßt :

– Verdichten von kristallinem Ibuprofen, wobei eine Aggregation des kristallinen Ibuprofens unter Bildung eines aggregierten Materials bewirkt wird,

– Vermahlen des aggregierten Materials, und

– Auswählen der Körnchen mit einer gewünschten Größe.

8. Verfahren nach Anspruch 7, worin die Schritte des Vermahlens des aggregierten Materials und Auswählens der Körnchen gleichzeitig erfolgen.

9. Verfahren nach einem der beiden Ansprüche 7 und 8, worin das aggregierte Material durch Sieben unter Herstellen der Körnchen vermahlen wird.

10. Verfahren nach irgendeinem der Ansprüche 7 bis 9, worin das kristalline Ibuprofen bis zu 50 Gew.-% einer Flüssigkeit enthält und die Flüssigkeit von dem aggregierten Material oder den Körnchen durch Trocknen entfernt wird.

11. Verfahren nach Anspruch 10, worin die Flüssigkeit einen Kohlenwasserstoff umfaßt.

12. Verfahren nach irgendeinem der Ansprüche 7 bis 11, worin das kristalline Ibuprofen verdichtet wird

(a) zwischen zwei in Gegenrichtung zueinander laufenden Walzen unter Bildung des aggregierten Materials ;

(b) anfänglich bei Durchlaufen zwischen einer ersten und einer zweiten Walze und danach bei Durchlaufen zwischen einer zweiten und einer dritten Walze einer Serie von drei Walzen, wobei die erste und dritte Walze so angeordnet sind, daß sie sich in derselben Richtung drehen und die zweite Walze so angeordnet ist, daß sie sich in Gegenrichtung dreht, und wobei das aggregierte Material nach Verdichten zwischen der zweiten und der dritten Walze gewonnen wird.

13. Verfahren nach irgendeinem der Ansprüche 10 bis 12, worin das kristalline Ibuprofen verdichtet wird durch

(a) Führen des kristallinen Ibuprofens zwischen zwei Walzen, wobei eine Walze eine feste Oberfläche aufweist und die andere Walze hohl ist und Extrudier-Öffnungen aufweist, die sich radial vom hohlen Innenbereich zur äußeren Oberfläche der Walze erstrecken, und Extrudieren des kristallinen Ibuprofens durch die Extrudier-Öffnungen in das Innere der Hohlwalze ;

(b) Transportieren des kristallinen Ibuprofens mittels Transportschnecken durch einen Extruder und Extrudieren des kristallinen Ibuprofens durch eine Extruder-Scheibe, wobei die Extruder-Scheibe Extrudier-Öffnungen umfaßt, die sich durch die gesamte Dicke der Scheibe erstrecken ; oder

(c) Kneten des kristallinen Ibuprofens mit Knetpaddeln, sobald es durch Transportschnecken durch einen Extruder unter Bildung eines aggregierten Materials transportiert wurde.

## Patentansprüche für folgenden Vertragsstaaten : AT, GR, ES

1. Verfahren zur Herstellung einer festen pharmazeutischen Zusammmensetzung, die Körnchen umfaßt, die im wesentlichen aus einem Aggregat von Kristallen aus Ibuprofen bestehen, worin die Kristalle eine mittlere Teilchengröße im Bereich von 5 bis 100 µm aufweisen und die Körnchen eine Porosität im Bereich von 4 bis 40 % aufweisen, wobei das Verfahren folgende Schritte umfaßt :

– Verdichten kristallinen Ibuprofens, wobei man eine Aggregation des kristallinen Ibuprofens unter Bildung eines aggregierten Materials herbeiführt,

– Vermahlen des aggregierten Materials und

– Auswählen der Körnchen mit einer gewünschten Größe.

2. Verfahren nach Anspruch 1, worin die Schritte des Vermahlens, des aggregierten Materials und des Auswählens der Körnchen gleichzeitig ablaufen.

3. Verfahren nach einem der beiden Ansprüche 1 und 2, worin das aggregierte Material durch Sieben unter Herstellung der Körnchen vermahlen wird.

4. Verfahren nach irgendeinem der Ansprüche 1 bis 3, worin das kristalline Ibuprofen bis zu 50 Gew.-% einer Flüssigkeit enthält und die Flüssigkeit von dem aggregierten Material oder den Körnchen durch Trocknen entfernt wird.

5. Verfahren nach Anspruch 4, worin die Flüssigkeit einen Kohlenwasserstoff umfaßt.

6. Verfahren nach irgendeinem der Ansprüche 1 bis 5, worin das kristalline Ibuprofen verdichtet wird

(a) zwischen zwei in Gegenrichtung zueinander laufenden Walzen unter Bildung des aggregierten Materials ;

(b) anfänglich bei Durchlaufen zwischen einer ersten und einer zweiten Walze und danach bei Durchlaufen zwischen einer zweiten und einer dritten Walze einer Serie von drei Walzen, wobei die erste und dritte Walze so angeordnet sind, daß sie sich in derselben Richtung drehen und die zweite Walze so angeordnet ist, daß sie sich in Gegenrichtung dreht, und wobei das aggregierte Material nach Verdichten zwischen der zweiten und der dritten Walze gewonnen wird.

7. Verfahren nach irgendeinem der Ansprüche 4 und 5, worin das kristalline Ibuprofen verdichtet wird durch

(a) Führen des kristallinen Ibuprofens zwischen zwei Walzen, wobei eine Walze eine feste Oberfläche aufweist und die andere Walze hohl ist und Extrudier-Öffnungen aufweist, die sich radial vom hohlen Innenbereich zur äußeren Oberfläche der Walze erstrecken, und extrudieren des kristallinen Ibuprofens durch die Extrudier-Öffnungen in das Innere der Hohlwalze ;

(b) Transportieren des kristallinen Ibuprofens mittels Transportschnecken durch einen Extruder und Extrudieren des kristallinen Ibuprofens durch eine Extruder-Scheibe, wobei die Extruder-Scheibe Extrudier-Öffnungen umfaßt, die sich durch die gesamte Dicke der Scheibe erstrecken ; oder

(c) Kneten des kristallinen Ibuprofens mit Knetpaddeln, sobald es durch Transportschnecken durch einen Extruder unter Bildung eines aggregierten Materials transportiert wurde.

8. Verfahren zur Herstellung einer festen pharmazeutischen Formulierung, welche 50 bis 95 Gew.-% einer Zusammensetzung umfaßt, die Körnchen umfaßt, die im wesentlichen aus einem Aggregat von Kristallen von Ibuprofen bestehen, worin die Kristalle eine mittlere Teilchengröße im Bereich von 5 bis 100 µm aufweisen und die Körnchen eine Porosität im Bereich von 4 bis 40 % aufweisen, zusammen mit einem pharmazeutisch annehmbaren Träger, worin die Zusammensetzung mit dem pharmazeutisch annehmbaren Träger vermischt und in Form fester Dosierungseinheiten verpresst wird.


## Revendications

**Revendications pour les Etats contractants suivants : BE, CH, DE, FR, GB, IT, LI, LU, NL, SE**

1. Composition pharmaceutique solide qui se compose de granules essentiellement constitués d'un agrégat de cristaux d'ibuprofène, caractérisée en ce que les cristaux possèdent un calibre moyen des particules qui varie de 5 à 100 µm et les granules possèdent une porosité qui fluctue de 4 à 40% en poids.

2. Composition suivant la revendication 1, caractérisée en ce que les granules possèdent une masse spécifique apparente qui varie de 0,3 à 0,7 g/cm³ et une masse spécifique à l'état tassé qui varie de 0,4 à 0,8 g/cm³.

3. Composition suivant l'une quelconque des revendications précédentes, caractérisée en ce que les granules possèdent un calibre moyen des particules qui fluctue de 50 à 2000 µm.

4. Composition pharmaceutique solide, caractérisée en ce qu'elle comprend de 50 à 95% en poids d'une composition suivant l'une quelconque des revendications 1 à 3, ainsi qu'un excipient ou véhicule pharmaceutiquement acceptable.

5. Composition suivant la revendication 4, caractérisée en ce que le véhicule ou excipient pharmaceutiquement acceptable se compose d'une ou plusieurs des substances suivantes : acide stéarique, cellulose ou dérivés de cellulose, disaccharides, dioxyde de silicium colloïdal, croscarmellose sodique, stéarate de magnésium, poudre de talc, phosphate tricalcique.

6. Composition suivant l'une quelconque des revendications 4 et 5, caractérisée en ce que les granules sont enrobés d'un excipient ou véhicule à libération soutenue de la substance médicamenteuse, de façon à former une composition à libération soutenue ou progressive de la substance médicamenteuse.

7. Procédé de préparation d'une composition pharmaceutique solide, caractérisée en ce qu'elle se compose de granules essentiellement constitués d'un agrégat de cristaux d'ibuprofène, dont les cristaux possèdent un calibre moyen des particules qui varie de 5 à 100 µm et les granules possèdent une porosité qui fluctue de 4 à 40%, caractérisé en ce que l'on tasse de l'ibuprofène cristallin de manière à provoquer une agrégation de l'ibuprofène cristallin en une forme de matière agrégée, on broie la matière agrégée et on choisit les granules qui possèdent le calibre voulu.

8. Procédé suivant la revendication 7, caractérisé en ce que le broyage de la matière agrégée et le choix des granules s'opèrent simultanément.

9. Procédé suivant l'une quelconque des revendications 7 et 8, caractérisé en ce que l'on broie la matière agrégée par criblage ou tamisage pour produire les granules.

10. Procédé suivant l'une quelconque des revendications 7 à 9, caractérisé en ce que l'ibuprofène cristallin contient jusqu'à 50% en poids d'un liquide et on élimine le liquide de la matière agrégée ou des granules par séchage.

11. Procédé suivant la revendication 10, caractérisé en ce que le liquide est constitué d'un hydrocarbure.

12. Procédé suivant l'une quelconque des revendications 7 à 11, caractérisé en ce que l'on tasse l'ibuprofène cristallin

a) entre des rouleaux tournant en sens contraire l'un par rapport à l'autre pour former la matière agrégée,

b) initialement, lorsqu'il passe entre un premier et un second rouleaux et, ensuite, lorsqu'il passe entre des second et troisième rouleaux d'un jeu de trois rouleaux, le premier et le troisième rouleaux étant agencés de manière à tourner dans la même direction, le second rouleau étant prévu pour tourner dans la direction opposée et on recueille matière agrégée après tassement entre les second et troisième rouleaux.

13. Procédé suivant l'une quelconque des revendications 10 et 12, caractérisé en ce que l'on tasse l'ibuprofène cristallin

a) en forçant l'ibuprofène cristallin à passer entre deux rouleaux, l'un des rouleaux possédant une surface pleine et l'autre rouleau étant creux et présentant des ouvertures d'extrusion qui s'étendent radialement à partir de l'intérieur creux jusqu'à la surface externe du rouleau et en extrudant l'ibuprofène cristallin à travers les ouvertures d'extrusion à l'intérieur du rouleau creux,

b) en transportant l'ibuprofène cristallin à l'aide de vis d'alimentation à travers une extrudeuse et en extrudant l'ibuprofène cristallin à travers une plaque d'extrusion, la plaque d'extrusion comprenant des ouvertures d'extrusion qui s'étendent à travers l'épaisseur de la plaque, ou

c) en malaxant l'ibuprofène cristallin à l'aide de pales ou palettes de malaxage au fur et à mesure qu'il est transporté par les vis d'alimentation à travers une extrudeuse pour former une matière agrégée.

**Revendications pour les Etats contractants suivants : AT, GR, ES**

1. Procédé de préparation d'une composition pharmaceutique solide, caractérisée en ce qu'elle se compose de granules essentiellement constitués d'un agrégat de cristaux d'ibuprofène, dont les cristaux possèdent un calibre moyen des particules qui varie de 5 à 100 µm et les granules possèdent une porosité qui fluctue de 4 à 40%, caractérisé en ce que l'on tasse de l'ibuprofène cristallin de manière à provoquer une agrégation de l'ibuprofène cristallin en une forme de matière agrégée, on broie la matière agrégée et on choisit les granules qui possèdent le calibre voulu.

2. Procédé suivant la revendication 1, caractérisé en ce que le broyage de la matière agrégée et le choix des granules s'opèrent simultanément.

3. Procédé suivant l'une quelconque des revendications 1 et 2, caractérisé en ce que l'on broie la matière agrégée par criblage ou tamisage pour produire les granules.

4. Procédé suivant l'une quelconque des revendications 1 à 3, caractérisé en ce que l'ibuprofène cristallin contient jusqu'à 50% en poids d'un liquide et on élimine le liquide de la matière agrégée ou des granules par séchage.

5. Procédé suivant la revendication 10, caractérisé en ce que le liquide est constitué d'un hydrocarbure.

6. Procédé suivant l'une quelconque des revendications 1 à 5, caractérisé en ce que l'on tasse l'ibuprofène cristallin

a) entre des rouleaux tournant en sens contraire l'un par rapport à l'autre pour former la matière agrégée,

b) initialement, lorsqu'il passe entre un premier et un second rouleaux et, ensuite, lorsqu'il passe entre des second et troisième rouleaux d'un jeu de trois rouleaux, le premier et le troisième rouleaux étant agencés de manière à tourner dans la même direction, le second rouleau étant prévu pour tourner dans la direction opposée et on recueille matière agrégée après tassement entre les second et troisième rouleaux.

7. Procédé suivant l'une quelconque des revendications 4 et 5, caractérisé en ce que l'on tasse l'ibuprofène cristallin

a) en forçant l'ibuprofène cristallin à passer entre deux rouleaux, l'un des rouleaux possédant une surface pleine et l'autre rouleau étant creux et présentant des ouvertures d'extrusion qui s'étendent radialement à partir de l'intérieur creux jusqu'à la surface externe du rouleau et en extrudant l'ibuprofène cristallin à travers les ouvertures d'extrusion à l'intérieur du rouleau creux,

b) en transportant l'ibuprofène cristallin à l'aide de vis d'alimentation à travers une extrudeuse et en extrudant l'ibuprofène cristallin à travers une plaque d'extrusion, la plaque d'extrusion comprenant des ouvertures d'extrusion qui s'étendent à travers l'épaisseur de la plaque, ou

c) en malaxant l'ibuprofène cristallin à l'aide de pales ou palettes de malaxage au fur et à mesure qu'il est transporté par les vis d'alimentation à travers une extrudeuse pour former une matière agrégée.

8. Procédé de préparation d'une composition pharmaceutique solide comprenant de 5 à 95% en poids d'une composition composée de granules essentiellement constitués d'un agrégat de cristaux d'ibuprofène, dont les cristaux possèdent un calibre moyen des particules qui varie de 5 à 100 µm et possèdent une porosité qui fluctue de 4 à 40%, ainsi qu'un véhicule ou excipient pharmaceutiquement acceptable, caractérisé en ce que l'on mélange la composition au véhicule ou excipient pharmaceutiquement acceptable et on comprime le mélange en une forme de dosage solide.